# EUROPEAN PATENT APPLICATION

(11) **EP 2 837 684 A1**
(43) Date of publication of application: **18.02.2015**
(21) Application number: 13774942.0
(22) Date of filing: 10.04.2013
(51) Int. Cl.: C12N 15/09, C12Q 1/68

(54) **METHOD FOR DETECTING TARGET NUCLEIC ACID USING MOLECULAR BEACON-TYPE PROBE**

(30) Priority: 11.04.2012 JP 2012090373
(71) Applicant: Fujirebio Inc., Shinjuku-ku Tokyo 163-0410 (JP)
(72) Inventor: MATSUNO, Tatsuki, Tokyo 163-0410 (JP)
(74) Representative: Hoffmann Eitle
(86) International application number: PCT/JP2013/060861
(87) International publication number: WO 2013/154138

(57) **Abstract**

The present invention provides a detection system using a nucleic acid probe which is commonly used in a measurement system using a recombinase (e.g., nucleic acid amplification reaction). Specifically, the present invention provides a method for detecting a target nucleic acid, comprising measuring the target nucleic acid using a cleavage enzyme, a recombinase, and a probe having characteristics of the following (a) to (c):
(a) being a single-stranded nucleic acid molecule comprising a pair of regions capable of complementarily binding to each other, and a region capable of complementarily binding to the target nucleic acid;
(b) comprising a site to be cleaved by the cleavage enzyme; and
(c) having a terminal label.

SEQUENCE LISTING

<110>  FUJIREBIO INC.

<120>  Method of detecting target nucleoic acid using molecular beacon
       type probe

<130>  PRBA-25305

<150>  JP 2012-090373
<151>  2012-04-11

<160>  3

<170>  PatentIn version 3.5

<210>  1
<211>  33
<212>  DNA
<213>  Artificial Sequence

<220>
<223>  Forward primer for amplifying a polynucleitide encoding S antigen
       of HBV

<400>  1
ctggctatcg ctggatgtgt ctgcggcgtt tta                                33


<210>  2
<211>  33
<212>  DNA
<213>  Artificial Sequence

<220>
<223>  Reverse primer for amplifying a polynucleitide encoding S antigen
       of HBV

<400>  2
ttgcgaaagc ccaggatgat gggatgggaa tac                                33


<210>  3
<211>  30
<212>  DNA
<213>  Artificial Sequence

<220>
<223>  Molecular beacon type probe for detecting a polynucleitide
       encoding S antigen of HBV


<220>
<221>  misc_feature
<222>  (1)..(11)
<223>  Nucleotide residues at positions 1 to 11 are DNA molecules

<220>
<221>  misc_feature
<222>  (12)..(12)
<223>  Nucleotide residue at position 12 is RNA molecule

<220>

<221>  misc_feature
<222>  (13)..(30)
<223>  Nucleotide residues at positions 13 to 30 are DNA molecules

<400>  3
ccgtcgttgc tgtacaaaac cttcggacgg                                        30

## Description

### TECHNICAL FIELD

The present invention relates to a method and kit for detecting a target nucleic acid.

### BACKGROUND ART

Nucleic acid amplification reactions associated with temperature change (e.g., PCR) and isothermal nucleic acid amplification reactions are available as nucleic acid amplification reactions. An RPA (recombinase polymerase amplification) method that is a method using a recombinase is known as the isothermal nucleic acid amplification reaction method. The nucleic acid amplification reaction is utilized to detect a target nucleic acid, and a molecular beacon probe having a stem portion and a loop portion and capable of forming a stem-loop structure (Patent Literature 1) and a cycling probe having a site to be cleaved by an RNase H (Patent Literature 2) are known as a probe for detecting a target nucleic acid in the nucleic acid amplification reaction. Also, a special nucleic acid probe, i.e., a nucleic acid probe that is internally labeled with a fluorescent substance and a quencher and has a site to be cleaved by a cleavage enzyme is used in the nucleic acid amplification reaction using a recombinase (Non-patent Literature 1).

### PRIOR ART REFERENCES

### PATENT LITERATURES

Patent Literature 1: WO1995/013399
Patent Literature 2: WO89/10415

### NON-PATENT LITERATURES

Non-patent Literature 1: Piepenburg et al, PLoS Biology 2006, Volume 4, Issue 7, e204

### DISCLOSURE OF INVENTION

### PROBLEM TO BE SOLVED BY THE INVENTION

In the nucleic acid amplification reaction using a recombinase, a probe that forms a stem-loop structure under a condition that is generally utilized in other nucleic acid amplification reactions cannot form that structure due to an influence of the recombinase. Therefore, in detection by the nucleic acid amplification reaction using the recombinase, a molecular beacon probe having a fluorescent substance and a quencher at its both termini, respectively, which is generally used as a detection system for the nucleic acid amplification reaction, cannot be used. Instead, the aforementioned special nucleic acid probe that is internally labeled with the fluorescent substance and the quencher and has the site to be cleaved by the cleavage enzyme is used (Non-patent Literature 1).

In order to synthesize a nucleic acid probe that does not have the fluorescent substance and the quencher at its both termini but has them internally, nucleotide residues internally present in the nucleic acid probe are necessary to be labeled with the fluorescent substance and the quencher. In the synthesis of such an internally labeled nucleic acid probe, a nucleic acid probe having functional groups such as amino groups is initially synthesized and subsequently these functional groups must be reacted with the fluorescent substance and the quencher. However, when the fluorescent substance and the quencher are reacted with the functional group, harsh conditions such as heating are required, and a substance to be labeled may be decomposed during the reaction. Therefore, types of labeling substances that can be used for such labeling are limited. Since special technologies are also required to synthesize such a nucleic acid probe, there are problems in its synthesis including difficulties in synthesis and high cost for synthesis.

Therefore, the object of the present invention is to establish a detection system using a versatile nucleic acid probe that can utilize various labeling substances in a measurement system using a recombinase (e.g., nucleic acid amplification reaction).

### MEANS FOR SOLVING PROBLEM

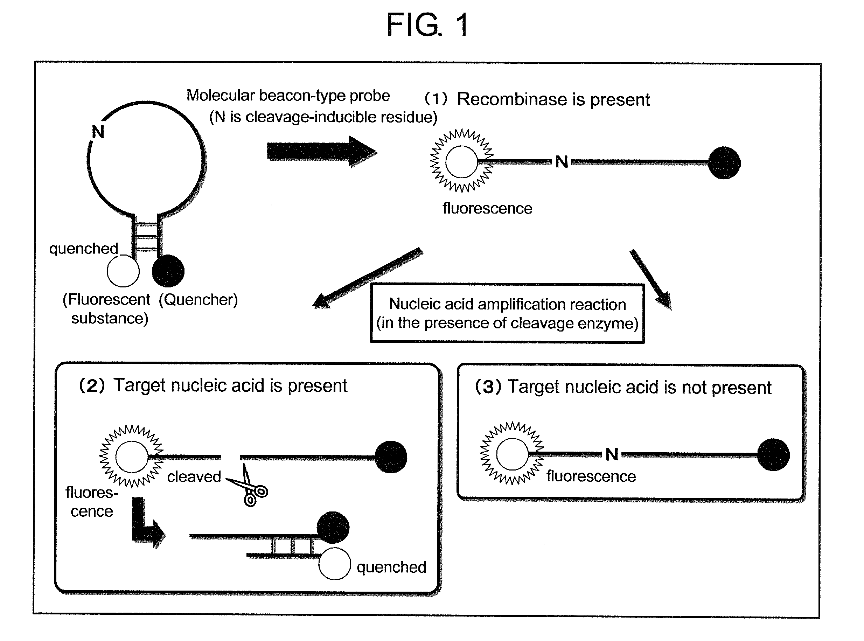

As a result of an extensive study, the present inventors have succeeded in establishing a novel detection system by taking advantage of the fact that a molecular beacon probe cannot form a stem-loop structure due to an influence of a recombinase under a measurement condition using a recombinase (FIG. 1). Thus, the present inventors have succeeded in solving the above problem and completed the present invention.

Accordingly, the present invention is as follows.
[1] A method for detecting a target nucleic acid, comprising measuring the target nucleic acid using a cleavage enzyme, a recombinase, and a probe having characteristics of the following (a) to (c):
   (a) being a single-stranded nucleic acid molecule comprising a pair of regions capable of complementarily binding to each other, and a region capable of complementarily binding to the target nucleic acid;
   (b) comprising a site to be cleaved by the cleavage enzyme; and
   (c) having a terminal label.
[2] The method according to [1], wherein the measurement of the target nucleic acid is performed over time.
[3] The method according to [1] or [2], wherein the measurement of the target nucleic acid is performed by the following (1) and (2):
   (1) subjecting a nucleic acid sample to a nucleic acid amplification reaction of the target nucleic acid using the recombinase in the presence of the probe and the cleavage enzyme; and
   (2) detecting a detection signal caused by the probe in the nucleic acid amplification reaction.
[4] The method according to [3], further comprising evaluating the presence or absence of the target nucleic acid or an amount of the target nucleic acid based on change of the detection signal over time.
[5] The method according to any of [1] to [4], wherein the probe comprises the site to be cleaved by the cleavage enzyme in the region capable of complementarily binding to the target nucleic acid.
[6] The method according to any of [1] to [5], wherein the probe is terminally labeled by the following (c1) or (c2): (c1) the probe has a detection signal-generating substance at 5'-terminus and has a substance capable of interfering with the detection signal-generating substance at 3'-terminus; or
(c2) the probe has a substance capable of interfering with a detection signal-generating substance at 5'-terminus and has the detection signal-generating substance at 3'-terminus.
[7] The method according to [6], wherein the detection signal-generating substance is a fluorescent substance and the substance capable of interfering with the detection signal-generating substance is a quencher.
[8] The method according to [7], wherein the amount of the target nucleic acid is measured by monitoring a decrease of fluorescence intensity over time in detection of the detection signal.
[9] The method according to any of [1] to [8], wherein the cleavage enzyme is an RNase H.
[10] The method according to any of [2] to [9], wherein the amplification reaction of the target nucleic acid is performed under a temperature condition of 60°C or lower.
[11] The method according to [10], wherein the amplification reaction of the target nucleic acid is performed under a temperature condition of 50°C or lower.
[12] The method according to any of [3] to [11], wherein the nucleic acid amplification reaction is an isothermal nucleic acid amplification reaction.
[13] The method according to [12], wherein the isothermal nucleic acid amplification reaction is an RPA method.
[14] A kit comprising a cleavage enzyme, a recombinase, and a probe having characteristics of the following (a) to (c):
   (a) being a single-stranded nucleic acid molecule comprising a pair of regions capable of complementarily binding to each other, and a region capable of complementarily binding to the target nucleic acid;
   (b) comprising a site to be cleaved by the cleavage enzyme; and
   (c) having a terminal label.
[15] The kit according to [14], further comprising a DNA polymerase.

### EFFECT OF THE INVENTION

The method of the present invention is useful because it can be performed using a probe that can utilize various labeling substances (e.g., detection signal-generating substances and substances capable of interfering with the detection signal-generating substance) and has advantages such as low synthesis difficulty and low synthesis cost.

### BRIEF DESCRIPTION OF DRAWINGS

FIG. 1 illustrates a principle for detection of a target nucleic acid by a nucleic acid amplification reaction using a recombinase in the presence of a molecular beacon-type probe and a cleavage enzyme. The principle is explained using a fluorescent substance as a detection signal-generating substance and a quencher as a substance capable of interfering with the detection signal-generating substance by way of example.
   (1) In the case where a recombinase is present: a molecular beacon-type probe itself cannot be directly used for detection of a target nucleic acid by a nucleic acid amplification reaction using a recombinase. This is because (i) a stem-loop structure cannot be formed due to an influence of the recombinase under condition of the nucleic acid amplification reaction using the recombinase and (ii) when the stem-loop structure cannot be formed, a distance between a fluorescent substance and a quencher becomes long. As a result, the fluorescent substance and the quencher cannot interact with each other, and thus fluorescence is not quenched.
   (2) In the case where a target nucleic acid is present: first, a molecular beacon-type probe (single-stranded nucleic acid) forms a double strand with the target nucleic acid. Then, the formed double strand is recognized by a cleavage enzyme and the molecular beacon-type probe is cleaved. Finally, two regions capable of complementarily binding to each other that are formed by the cleavage are hybridized to form a duplex, and thus fluorescence is quenched.
   (3) In the case where a target nucleic acid is not present: a molecular beacon-type probe does not form a double strand with the target nucleic acid. Consequently, the molecular beacon-type probe is not cleaved, and thus fluorescence is not quenched.
FIG. 2 illustrates changes of fluorescent intensity in a nucleic acid amplification reaction of a target nucleic acid using a recombinase in the presence of a molecular beacon-type probe and an enzyme having an RNase H activity. HBV DNA: hepatitis B virus DNA; DW: distilled water.
FIG. 3 illustrates quenching of fluorescence signals depending on amounts of added target nucleic acid by the method of the present invention. Various copy numbers of HBV DNA were used as the target nucleic acid.
FIG. 4 illustrates changes of fluorescent intensity relative to temperature changes in RPA reaction and PCR reaction using a molecular beacon-type probe.

### EMBODIMENTS FOR CARRYING OUT THE INVENTION

The present invention provides a method for detecting a target nucleic acid.

The method of the present invention comprises measuring the target nucleic acid using a probe, a cleavage enzyme and a recombinase.

The probe used in the method of the present invention can have the following characteristics (a) to (c):
(a) being a single-stranded nucleic acid molecule comprising a pair of regions capable of complementarily binding to each other and a region capable of complementarily binding to a target nucleic acid;
(b) comprising a site to be cleaved by a cleavage enzyme; and
(c) having a terminal label.

The characteristic (a) is described.

The probe used in the method of the present invention can be a molecular beacon-type probe having a structure that is similar to that of a molecular beacon probe having a stem portion and a loop portion (the probe used in the method of the present invention may be referred to as molecular beacon-type probe). Specifically, the probe used in the method of the present invention can be a single-stranded nucleic acid molecule comprising a pair of regions capable of complementarily binding to each other, which can correspond to the stem portion of the molecular beacon probe, and a region capable of complementarily binding to a target nucleic acid, which can correspond to the loop portion of the molecular beacon probe. The probe used in the method of the present invention can form a double strand with the target nucleic acid, since it comprises the region capable of complementarily binding to the target nucleic acid as described above.

The probe used in the method of the present invention comprises nucleotide residues (DNA or RNA) having nucleobases selected from the group consisting of adenine (A), guanine (G), cytosine (C), uracil (U) and thymine (T), and modified nucleobases thereof (e.g., tetrahydrofuran residue, inosine, uridine, 3-nitropyrrol, 5-nitroindole, pyrimidine derivatives, purine derivatives).

A full length of the probe used in the method of the present invention is a nucleotide length of, for example, 23 or more, preferably 26 or more and more preferably 32 or more. The full length of the probe used in the method of the present invention is also a nucleotide length of, for example, 100 or less, preferably 80 or less and more preferably 60 or less.

The one pair of the regions capable of complementarily binding to each other is composed of any nucleotide residues that enable complementary intramolecular binding, and, for example, composed of one type or more (e.g., 1, 2, 3 or 4 types) of the aforementioned nucleotide residues that are DNA (e.g., A, G, C and T that are typical components of DNA). It is preferable that the one pair of the regions capable of complementarily binding to each other has no cleavage-inducible residue described later. It is preferable that each region in the one pair of the regions capable of complementarily binding to each other is present in the 5'-terminal region and the 3'-terminal region of the probe. It is preferable that the regions capable of complementarily binding to each other are designed so as not to complementarily bind to the target nucleic acid.

A length of each region in the one pair of the regions capable of complementarily binding to each other is a nucleotide length of, for example, 3 or more, preferably 5 or more and more preferably 6 or more. The length of the one pair of the regions capable of complementarily binding to each other is also a nucleotide length of, for example, 25 or less, preferably 20 or less and more preferably 15 or less.

The region capable of complementarily binding to the target nucleic acid comprises any nucleotide residues that enables binding between the probe and the target nucleic acid, or that enables complementary binding, and, for example, comprises one type or more (e.g., 1, 2, 3 or 4 types) of the aforementioned nucleotide residues that are DNA (e.g., A, G, C and T that are typical components of DNA). It is preferable that the region capable of complementarily binding to the target nucleic acid is present between the one pair of the regions capable of complementarily binding to each other. In addition, it is preferable that the region capable of complementarily binding to the target nucleic acid has one or more (e.g., 1, 2, 3 or 4) of the cleavage-inducible residues described later. The cleavage-inducible residue in the region capable of complementarily binding to the target nucleic acid may or may not be complementarily bound to a certain nucleotide in the target nucleic acid. The cleavage-inducible residue may be inserted in a position of the 1st, 2nd, 3rd, 4th, 5th, 6th, 7th, 8th, 9th, 10th, 11th, 12th, 13th, 14th, 15th, 16th, 17th, 18th, 19th, or 20th nucleotide residue counting from one of terminal nucleotide residues of the region capable of complementarily binding to the target nucleic acid (1st nucleotide residue in the region capable of complementarily binding to the target nucleic acid).

A length of the region capable of complementarily binding to the target nucleic acid is a nucleotide length of, for example, 20 or more and preferably 30 or more. The length of the region capable of complementarily binding to the target nucleic acid is also a nucleotide length of, for example, 50 or less and preferably 40 or less. In terms of recognition by the recombinase, the length of the region capable of complementarily binding to the target nucleic acid is preferably a nucleotide length of 20 to 50, and more preferably a nucleotide length of 20 to 40, and further preferably a nucleotide length of 30 to 34.

The characteristic (b) is described.

The probe used in the method of the present invention can also comprise a site to be cleaved by the cleavage enzyme.

The "cleavage enzyme" in the present invention means an enzyme having an activity to cleave the probe used in the present invention (single-stranded nucleic acid) alone in two nucleic acids that form a double-stranded nucleic acid composed of the probe and a target nucleic acid when the probe is complementarily bound to the target nucleic acid to form the double-stranded nucleic acid. Therefore, the cleavage enzyme cannot have an activity to cleave the target nucleic acid in the two nucleic acids that form the double-stranded nucleic acid composed of the probe used in the present invention (single-stranded nucleic acid) and the target nucleic acid when the probe is complementarily bound to the target nucleic acid to form the double-stranded nucleic acid. Examples of the cleavage enzyme may include enzymes having an RNase H activity. The "enzyme having the RNase H activity" in the present invention refers to an enzyme having an ability to cleave not only an RNA strand in a hybrid double-stranded nucleic acid composed of a DNA strand/an RNA strand but also a cleavage-inducible residue-containing DNA strand in a double-stranded nucleic acid composed of a DNA strand/the cleavage-inducible residue-containing DNA strand. Specifically, examples of the cleavage enzyme may include an RNase H, an exonuclease III, and an Nfo (e.g., derived from *E. coli).*

The "cleavage-inducible residue" in the present invention refers a residue that is recognized by the aforementioned cleavage enzyme to elicit cleavage of a single-stranded nucleic acid which contains a cleavage-inducible residue when the single-stranded nucleic acid is complementarily bound to the target nucleic acid to form a double-stranded nucleic acid. The cleavage-inducible residues do not include A, G, C and T that are typical components of DNA (i.e., 2'-deoxyadenosine 5'-phosphate, 2'-deoxyguanosine 5'-phosphate, 2'-deoxycytidine 5'-phosphate, and 2'-deoxythymidine 5'-phosphate). Examples of the cleavage-inducible residue may include constituent units of RNA (nucleotides having ribose as a sugar moiety) and a tetrahydrofuran residue (generally referred to as a d spacer). Examples of the constituent unit of RNA may include A, G, C and U that are typical components of RNA (i.e., adenosine 5'-phosphate, guanosine 5'-phosphate, cytidine 5'-phosphate, and uridine 5'-phosphate).

The probe comprising the site to be cleaved by the cleavage enzyme can be a chimeric single-stranded DNA comprising the cleavage-inducible residue as described above. The chimeric single-stranded DNA in the present invention refers to a single-stranded nucleic acid molecule having one type or more (e.g., 1, 2, 3 or 4 types) nucleotide residues selected from the group consisting of the aforementioned A, G, C and T that are typical components of DNA, and having at least one cleavage-inducible residue.

The characteristic (c) is described.

The probe used in the method of the present invention can further have a terminal label. The terminal label refers to that the probe that is a single-stranded nucleic acid molecule has a labeling substance at its terminus. The labeling substance means a substance that enables detection of a compound labeled with the labeling substance, and examples of the labeling substance may include detection signal-generating substances and substances capable of interfering with the detection signal-generating substance. The detection signal-generating substance is a compound capable of generating a detectable signal. Examples of the detection signal-generating substance may include fluorescent substances. The substance capable of interfering with the detection signal-generating substance is a compound that interacts with the detection signal-generating substance to change a detection signal. Examples of such a change of the detection signal may include disappearance and generation of the detection signal, and wavelength shift of the detection signal. Examples of the substance capable of interfering with the detection signal-generating substance may include quenchers and nucleic acid molecules exhibiting a fluorescence-quenching phenomenon.

The probe used in the method of the present invention may be terminally labeled by the following (c1) or (c2):
(c1) the probe has the detection signal-generating substance at 5'-terminus and has the substance capable of interfering with the detection signal-generating substance at 3'-terminus; or
(c2) the probe has the substance capable of interfering with the detection signal-generating substance at 5'-terminus and has the detection signal-generating substance at 3'-terminus.

In one embodiment, when the detection signal-generating substance is a fluorescent substance, the substance capable of interfering with the detection signal-generating substance is a quencher. Examples of the fluorescent substance used in this embodiment may include 6-FAM (6-carboxyfluorescein), TET (tetrachloro-6-carboxyfluorescein), HEX (hexachlorofluorescein), 6-JOE (6-carboxy-4',5'-dichloro-2',7'-dimethoxyfluorescein), ROX (carboxy-6-rhodamine) and TAMRA ((6-tetramethylrhodamine-5(6)-carboxamide)hexanoate). Examples of the quencher used in this embodiment may include BHQ-1 ([(4-(2-nitro-4-methyl-phenyl)-azo)-yl-((2-methoxy-5-methyl-phenyl)-azo)]-aniline), BHQ-2 (([(4-(1-nitro-phenyl)-azo)-yl-((2,5-dimethoxy-phenyl)-azo)]-aniline), Dabcyl (4-[[4-(dimethylamino)-phenyl]-azo]-benzoic acid), Eclipse (4-[[2-chloro-4-nitro-phenyl]-azo]-aniline), and TAMRA ((6-tetramethylrhodamine-5(6)-carboxamide)hexanoate).

In another embodiment, when the detection signal-generating substance is a fluorescent substance, the substance capable of interfering with the detection signal-generating substance may be a nucleic acid molecule exhibiting a fluorescence-quenching phenomenon. Examples of the fluorescent substance used in this embodiment (a fluorescent substance having a nature of being quenched by its interaction with the nucleic acid molecule exhibiting the fluorescence-quenching phenomenon) may include fluorescent substances that reduce a fluorescence signal when when positioned adjacent to a guanine base. Examples of the fluorescent substances that reduce the fluorescence signal when when positioned adjacent to the guanine base may include Pacific blue (2-oxo-6,8-difluoro-7-hydroxy-2H-1-benzopyran-3-carboxylic acid), BODIPY FL (N-[2-(iodoacetylamino)ethyl]-5,7-dimethyl-4,4-difluoro-3a-azonia-4-bora(IV)-4H-4a-aza-s-indacene-3-propaneamide), 5-CR6G (5-carboxyrhodamine), TAMRA ((6-tetramethylrhodamine-5(6)-carboxamide)hexanoate), 6-JOE (6-carboxy-4',5'-dichloro-2',7'-dimethoxyfluorescein), Alexa 488, Alexa 532, BODIPY TMR, and BODIPY FL/C3. The nucleic acid molecules exhibiting the fluorescence-quenching phenomenon, which is used in this embodiment, may include nucleic acid molecules having the guanine base (e.g., DNA, RNA).

As the recombinase, any recombinase that can be utilized for measurement of a target nucleic acid can be used, and a recombinase that can be utilized for a nucleic acid amplification reaction of a target nucleic acid using a recombinase is preferred. Examples of the recombinase that can be utilized in the method of the present invention may include UvsX, RecA and analogs thereof.

The measurement of the target nucleic acid may be performed over time. The measurement of the target nucleic acid over time means that the target nucleic acid is measured at different two or more time points. Therefore, the measurement of the target nucleic acid can be performed on a real-time basis. Examples of the different two or more time points may include different 2, 3, 4, 5, 6, 7, 8, 9 and 10 time points as well as more time points than them (e.g., 15, 20, 25, 30, 35, 40, 45 and 50 time points). A measurement interval that is a time interval between the above time points may or may not be constant, and is preferably constant. The measurement interval may be shorter than one minute (e.g., 10 seconds, 15 seconds, 20 seconds, 30 seconds, 40 seconds, or 50 seconds), or one minute or longer (e.g., 1 minute, 1 minute and 30 seconds, 2 minutes, 2 minutes and 30 seconds, 3 minutes, 4 minutes, or 5 minutes), and can be appropriately designed depending on a measurement purpose of the target nucleic acid.

The method of the present invention may be performed by the following (1) and (2):
(1) subjecting a nucleic acid sample to a nucleic acid amplification reaction of a target nucleic acid using a recombinase in the presence of a probe and a cleavage enzyme; and
(2) detecting a detection signal caused by the probe in the nucleic acid amplification reaction.

As the nucleic acid sample, a nucleic acid sample itself such as DNA or RNA, or any sample containing the same is used. The nucleic acid sample is not particularly limited, and may be a sample containing or suspected of containing a nucleic acid component derived from, for example, an animal such as a mammal, a plant, an insect, a microorganism (e.g., bacterium or a yeast), or a virus (e.g., a hepatitis virus such as HAV, HBV or HCV, or a retrovirus such as HIV). Examples of the mammal may include primates (e.g., human, monkey and chimpanzee), rodents (e.g., mouse, rat and guinia pig), pets (e.g., dog, cat and rabbit), working animals and domestic animals (e.g., cattle, horse, swine, sheep and goat). The nucleic acid sample may be a biological sample (e.g., blood, saliva, hair, mucosa, tissue sample) derived from a mammal suffering from or suspected of suffering from a disease. The nucleic acid sample is used in the method of the present invention after extracting a nucleic acid as needed. When a subject to be measured is DNA, the nucleic acid sample can be directly used in the method of the present invention. When a subject to be measured is RNA, the nucleic acid sample after a reverse transcription reaction can be used in the method of the present invention.

The nucleic acid amplification reaction using the recombinase can be performed under any temperature condition, and may be performed at temperature of about 60°C or lower, for example, a temperature of not more than about 55°C, about 50°C, about 45°C or about 40°C. The nucleic acid amplification reaction using the recombinase may also be performed at a temperature of about 25°C or higher, for example, a temperature of not less than about 30°C, about 37°C or about 40°C.

The nucleic acid amplification reaction using the recombinase may also be an isothermal nucleic acid amplification reaction. The isothermal nucleic acid amplification reaction can be generally classified into an isothermal nucleic acid amplification reaction performed under a moderate or high temperature condition at 50°C or higher (e.g., 50°C or higher and lower than 70°C) and an isothermal nucleic acid amplification reaction performed under a low temperature condition at 50°C or lower (e.g., 25°C or higher and 50°C or lower). The nucleic acid amplification reaction under an isothermal condition using the recombinase may be performed at low temperature of, for example, about 50°C or lower (e.g., a temperature of not more than about 45°C or about 40°C). The nucleic acid amplification reaction under the isothermal condition using the recombinase may be performed at a temperature of about 25°C or higher, for example, a temperature of not less than about 30°C, about 35°C or about 37°C.

The nucleic acid amplification reaction using the recombinase may be a reaction under the isothermal condition. Examples of the nucleic acid amplification reaction under the isothermal condition using the recombinase may include RPA (recombinase polymerase amplification) method and SIBA (strand invasion based amplification) method.

A concentration of the probe in a reaction solution used in the method of the present invention (e.g., nucleic acid amplification reaction) is, for example, 0.01 to 2 µM, preferably 0.02 to 1 µM, and more preferably 0.04 to 0.5 µM.

A concentration of the cleavage enzyme and the recombinase in the reaction solution used in the method of the present invention (e.g., nucleic acid amplification reaction) can be appropriately adjusted.

The other conditions of the nucleic acid amplification reaction (e.g., primer concentrations, an amount of a nucleic acid sample added to a reaction solution, and a reaction time) can be appropriately designed.

The detection of the detection signal can be performed by any method publicly known in the art. When a target nucleic acid is present, first a probe (single-stranded nucleic acid) is complementarily bound to the target nucleic acid to form a double-stranded nucleic acid composed of the probe and the target nucleic acid. Next the formed double-stranded nucleic acid is recognized by a cleavage enzyme and the probe is cleaved in the two nucleic acids forming the double-stranded nucleic acid. Finally, two separated regions capable of complementarily binding to each other, which is produced by the cleavage, are hybridized to form a duplex, thereby a detection signal-generating substance and a substance capable of interfering with the detection signal generating-substance are positioned adjacent to each other and interact to cause a change of the detection signal. The target nucleic acid can be measured by detecting the changed detection signal. Since such a detection signal is caused by the cleavage of the probe by the cleavage enzyme, degree of the detection signal change can be proportional to an amount of the probe cleaved by the cleavage enzyme. The amount of the probe cleaved by the cleavage enzyme can be proportional to an amount of the target nucleic acid capable of forming the double-stranded nucleic acid with the probe. In other words, the degree of the detection signal change can be proportional to the amount of the target nucleic acid. For example, when a fluorescent substance is used as the detection signal-generating substance and a quencher or a nucleic acid molecule exhibiting a fluorescence-quenching phenomenon is used as the substance capable of interfering with the detection signal-generating substance, degree of quenching of the fluorescence can be proportional to the amount of the target nucleic acid.

The detection of the detection signal may be performed over time. The detection of the detection signal over time means that the detection signal is detected at different two or more time points. Therefore, the detection of the detection signal can be performed on a real-time basis. Examples of the different two or more time points may include different 2, 3, 4, 5, 6, 7, 8, 9 and 10 time points as well as more time points than them (e.g., 15, 20, 25, 30, 35, 40, 45 and 50 time points). A measurement interval that is a time interval between the above time points may or may not be constant, and is preferably constant. The measurement interval may be shorter than one minute (e.g., 10 seconds, 15 seconds, 20 seconds, 30 seconds, 40 seconds, or 50 seconds), or one minute or longer (e.g., 1 minute, 1 minute and 30 seconds, 2 minutes, 2 minutes and 30 seconds, 3 minutes, 4 minutes, or 5 minutes), and can be appropriately designed depending on a measurement purpose of the target nucleic acid.

When the detection of the detection signal is performed over time, a change of the detection signal over time (e.g., decrease or increase) may be monitored. As a result of such a monitoring, the presence or absence of the target nucleic acid or the amount of the target nucleic acid can be evaluated based on the change of the detection signal over time. The method of the present invention may comprise such an evaluating step. For example, the method of the present invention may comprise such an evaluating step in addition to the above steps (1) and (2). Specifically, when a value of the detection signal is not substantially changed from a baseline level over time, it can be evaluated that the target nucleic acid is not present. On the other hand, when a value of the detection signal is substantially changed from the baseline level over time, it can be evaluated that the target nucleic acid is present. In addition, the amount of the target nucleic acid can be evaluated based on, for example, a time period until the value of the detection signal is substantially changed from the baseline level over time and/or changed degree of the detection signal per unit time after the value of the detection signal is substantially changed from the baseline level. Whether the value of the detection signal is substantially changed from the baseline level over time or not can be determined by, for example, comparing the value of the detection signal obtained by the method of the present invention with a value of the detection signal over time that is obtained by a negative control described later. The value of the detection signal obtained by the method of the present invention may also be compared with a value of the detection signal over time that is obtained using a positive control (e.g., it may be a serially diluted solutions of the target nucleic acid for preparing a standard curve) as described later.

For example, when a fluorescent substance is used as the detection signal-generating substance and a quencher or a nucleic acid molecule exhibiting a fluorescence-quenching phenomenon is used as the substance capable of interfering with the detection signal-generating substance, the presence or absence of the target nucleic acid or the amount of the target nucleic acid can be evaluated based on a decrease of fluorescence intensity over time (i.e., quenching of fluorescence). Specifically, when the detection intensity is not substantially decreased from the baseline level over time, it can be evaluated that the target nucleic acid is not present. On the other hand, when the detection intensity is substantially decreased from the baseline level over time, it can be evaluated that the target nucleic acid is present. In addition, the amount of the target nucleic acid can be evaluated based on, for example, a time period until the fluorescent intensity is substantially decreased from the baseline level and/or decreased degree of the fluorescence intensity per unit time after the fluorescent intensity is substantially decreased from the baseline level.

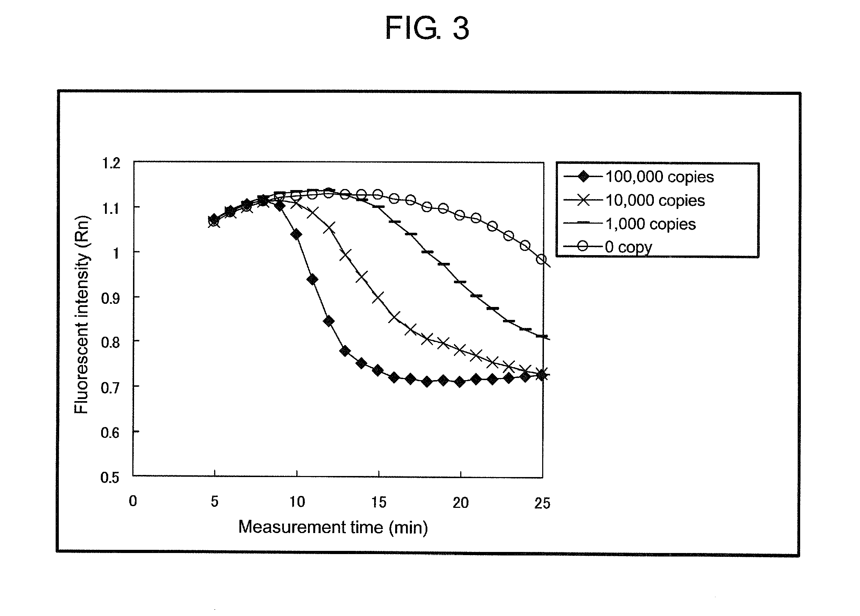

The method of the present invention is useful for the measurement of the target nucleic acid. For example, the present invention is entirely useful for qualitative assays (e.g., analyses of polymorphism such as SNP and haplotype) and quantitative assays (e.g., analyses of amounts of expressed genes or contents of genome in samples), and can be suitably used for the quantitative assays as demonstrated in Example 2 and FIG. 3. The method of the present invention is also useful for diagnoses, therapeutic monitoring and the like of animals such as humans.

The present invention also provides a kit.

The kit of the present invention comprises the aforementioned probe, cleavage enzyme and recombinase. The probe, the cleavage enzyme and the recombinase are as described above. The kit of the present invention is used, for example, for the aforementioned measurement system (e.g., nucleic acid amplification reaction).

The kit of the present invention may comprise a DNA polymerase.

The kit of the present invention may also comprise a single-stranded DNA-binding protein (SSB). The kit of the present invention may comprise dNTPs. The kit of the present invention may comprise ATP.

The kit of the present invention may also comprise primers for the target nucleic acid. The number of the primers of primers comprised in the kit of the present invention is, for example, two (RPA method) or three (SIBA method).

The kit of the present invention may also comprise a positive control [e.g., normal target nucleic acid (e.g., DNA or RNA)] and/or a negative control [e.g., solution containing no target nucleic acid or abnormal target nucleic acid (e.g., DNA or RNA)]. The kit of the present invention may further comprise another control such as a nucleic acid (e.g., DNA or RNA) for a housekeeping gene (e.g., GAPDH, β-actin, β2-microgloburin or HPRT1) that can be used as a comparative control in a qualitative assay and/or a quantitative assay. The kit of the present invention may also comprise primers for these controls. The kit of the present invention may comprise each component in an isolated form in an individual container (e.g., tube), or two or more components may be mixed previously in a single container.

### EXAMPLES

The present invention will be described in detail with reference to following examples, but the present invention is not limited by these Examples.

### Example 1: Detection of target nucleic acid by nucleic acid amplification reaction utilizing recombinase, molecular beacon-type probe and cleavage enzyme

TwistAmp Basic Kit based on an RPA method which is from TwistDx was used for a nucleic acid amplification assay. This kit comprises a DNA polymerase, a recombinase (UvsX), dNTPs, ATP and a single-stranded DNA-binding protein (SSB) as components required for the nucleic acid amplification reaction. Thus, these components were utilized in an assay shown below. DNA encoding a gene corresponding to an S-antigen site of HBV was used as a target nucleic acid. The forward primer: 5'-CTGGCTATCGCTGGATGTGTCTGCGGCGTTTTA-3' (SEQ ID NO:1) and the reverse primer: 5'-TTGCGAAAGCCCAGGATGATGGGATGGGAATAC-3' (SEQ ID NO:2) were used for amplification of the target nucleic acid. The sequence: 5'-CCGTCGTTGCT[G]TACAAAACCTTCGGACGG-3' (SEQ ID NO:3, G in the parenthesis is RNA) was used as a sequence of a molecular beacon-type probe that was a probe for detection. This sequence was labeled with 6-FAM (fluorescent substance) at 5'-terminus and with Eclipse (quencher) at 3'-terminus.

The forward primer, the reverse primer, the molecular beacon-type probe and ribonuclease H were added to rehydration buffer at final concentrations of 0.42 µM, 0.42 µM, 0.1 µM, and 1 U, respectively. Subsequently, the target nucleic acid (HBV DNA) was added at 10⁵ copies/50 µL, and 47.5 µL of the prepared solution was added to RPA pellet and then dissolved. Likewise, a solution using distilled water (DW) in place of the target nucleic acid was prepared. 2.5 µL of a solution of 280 mM magnesium acetate was added to the solution prepared above, and the mixture was stirred thoroughly and then incubated at 37°C for 25 minutes. A fluorescence signal was measured every one minute.

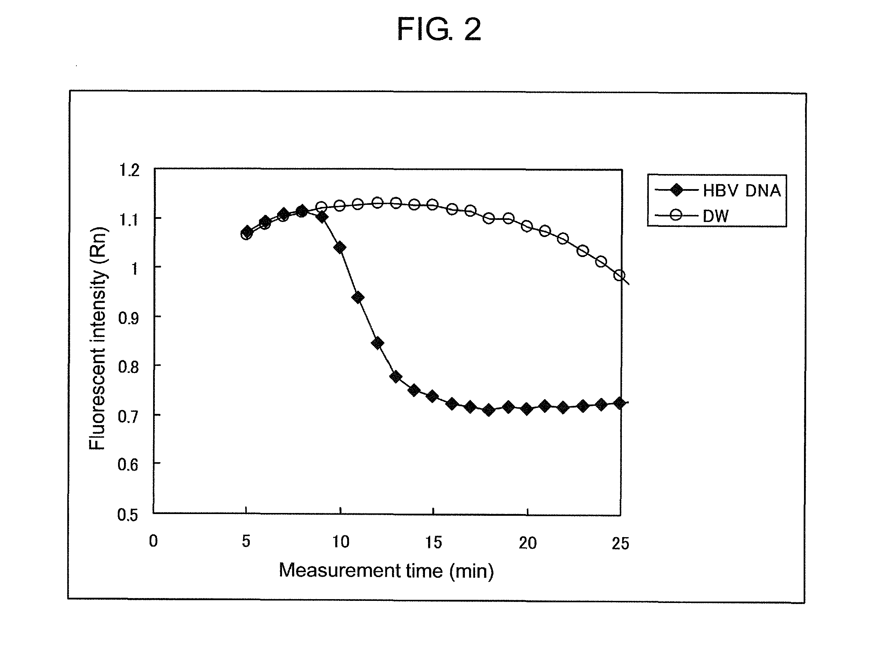

As a result, it was confirmed that the fluorescence signal was quenched when the target nucleic acid was present in the nucleic acid amplification reaction utilizing the recombinase, the molecular beacon-type probe and the cleavage enzyme (FIG. 2).

### Example 2: Examination of amount of target nucleic acid to be added

An experiment was performed in the same manner as in Example 1, except that an amount of the target nucleic acid (HBV DNA) to be added was changed to 10³ copies/50 µL, 10⁴ copies/50 µL or 10⁵ copies/50 µL. Likewise, the solution using distilled water (DW) in place of the target nucleic acid (0 copy) was also examined.

As a result, it was confirmed that a time at which the fluorescence signal was quenched was changed in a manner dependent on the amount of the added target nucleic acid (FIG. 3). Thus, it was shown that the method of the present invention can be used for quantification of the target nucleic acid.

### Example 3: Behavior of molecular beacon-type probe in temperature change under RPA reaction condition

To the rehydration buffer, the molecular beacon-type probe and magnesium acetate were added at final concentrations of 0.1 µM and 14 mM, respectively. Then, 50 µL of the prepared solution was added to RPA pellet and then dissolved. The solution prepared above was incubated at temperature rising from 37°C to 80°C at 0.5°C/30 seconds. The fluorescence signal was measured at each temperature during the incubation.

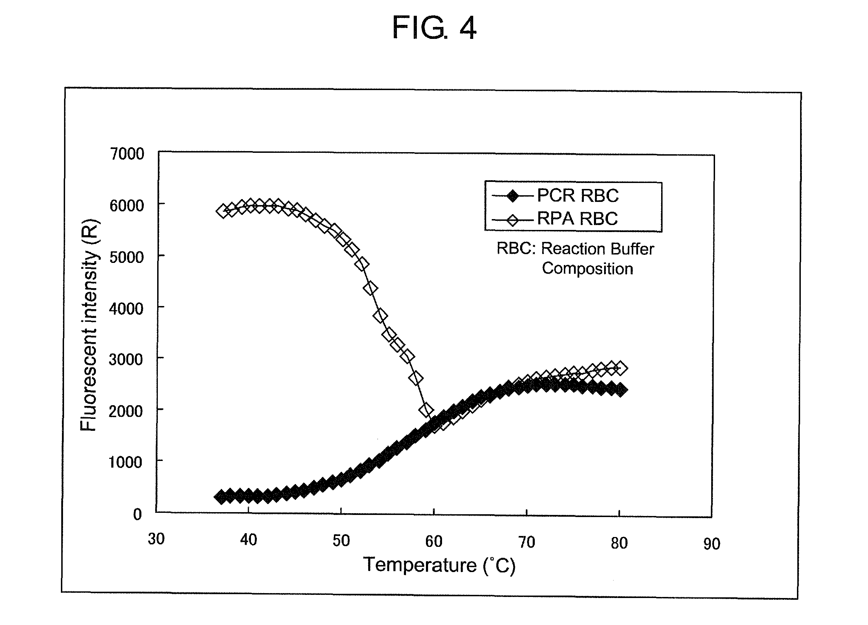

As a result, it was observed that the detected fluorescence intensity was high up to around 50°C, was diminished at a temperature of from 50°C to 60°C, and was reversed at temperature around 60°C (see "RPA RBC (reaction buffer composition)" in FIG. 4), indicating that the molecular beacon-type probe does not form a stem-loop structure at temperature of about 50°C or lower and that an effect of the recombinase is lost at a temperature of 50°C to 60°C in a temperature-dependent manner and the molecular beacon-type probe gradually forms the stem-loop structure. The reverse of the fluorescence intensity at temperature around 60°C indicates that the molecular beacon-type probe gets less able to keep the stem-loop structure according to temperature elevation.

### Reference Example 1: Behavior of molecular beacon-type probe in temperature change under PCR reaction condition

The molecular beacon-type probe was added at a final concentration of 0.1 µM to PCR buffer from ABI (10 mM Tris-HCl (pH 8.3), 50 mM KCl, 1.5 mM MgCl₂, and 0.001% gelatin).

The solution prepared above was incubated at temperature rising from 37°C to 80°C at 0.5°C/30 seconds. The fluorescence signal was measured at each temperature during the incubation.

As a result, it was observed that the fluorescence intensity was low up to temperature around 50°C and was increased according to temperature elevation, indicating that the molecular beacon-type probe forms the stem-loop structure at temperature up to around 50°C (see "PCR RBC (reaction buffer composition)" in FIG. 4).

### INDUSTRIAL APPLICABILITY

The present invention is useful for measurement of a target nucleic acid. For example, the present invention is useful for qualitative and quantitative assays as well as diagnoses and therapeutic monitoring of animals such as humans, and the like.

## Claims

1. A method for detecting a target nucleic acid, comprising measuring the target nucleic acid using a cleavage enzyme, a recombinase, and a probe having characteristics of the following (a) to (c):
(a) being a single-stranded nucleic acid molecule comprising a pair of regions capable of complementarily binding to each other, and a region capable of complementarily binding to the target nucleic acid;
(b) comprising a site to be cleaved by the cleavage enzyme; and
(c) having a terminal label.

2. The method according to claim 1, wherein the measurement of the target nucleic acid is performed over time.

3. The method according to claim 1 or 2, wherein the measurement of the target nucleic acid is performed by the following (1) and (2):
(1) subjecting a nucleic acid sample to a nucleic acid amplification reaction of the target nucleic acid using the recombinase in the presence of the probe and the cleavage enzyme; and
(2) detecting a detection signal caused by the probe in the nucleic acid amplification reaction.

4. The method according to claim 3, further comprising evaluating the presence or absence of the target nucleic acid or an amount of the target nucleic acid based on change of the detection signal over time.

5. The method according to any one of claims 1 to 4, wherein the probe comprises the site to be cleaved by the cleavage enzyme in the region capable of complementarily binding to the target nucleic acid.

6. The method according to any one of claims 1 to 5, wherein the probe is terminally labeled by the following (c1) or (c2):
(c1) the probe has a detection signal-generating substance at 5'-terminus and has a substance capable of interfering with the detection signal-generating substance at 3'-terminus; or
(c2) the probe has a substance capable of interfering with a detection signal-generating substance at 5'-terminus and has the detection signal-generating substance at 3'-terminus.

7. The method according to claim 6, wherein the detection signal-generating substance is a fluorescent substance and the substance capable of interfering with the detection signal-generating substance is a quencher.

8. The method according to claim 7, wherein the amount of the target nucleic acid is measured by monitoring a decrease of fluorescence intensity over time in detection of the detection signal.

9. The method according to any one of claims 1 to 8, wherein the cleavage enzyme is an RNase H.

10. The method according to any one of claims 2 to 9, wherein the amplification reaction of the target nucleic acid is performed under a temperature condition of 60°C or lower.

11. The method according to claim 10, wherein the amplification reaction of the target nucleic acid is performed under a temperature condition of 50°C or lower.

12. The method according to any one of claims 3 to 11, wherein the nucleic acid amplification reaction is an isothermal nucleic acid amplification reaction.

13. The method according to claim 12, wherein the isothermal nucleic acid amplification reaction is an RPA method.

14. A kit comprising a cleavage enzyme, a recombinase, and a probe having characteristics of the following (a) to (c):
(a) being a single-stranded nucleic acid molecule comprising a pair of regions capable of complementarily binding to each other, and a region capable of complementarily binding to the target nucleic acid;
(b) comprising a site to be cleaved by the cleavage enzyme; and
(c) having a terminal label.

15. The kit according to claim 14, further comprising a DNA polymerase.
